(19) Europäisches Patentamt / European Patent Office / Office européen des brevets

(11) **EP 3 265 929 B1**

(12) **EUROPEAN PATENT SPECIFICATION**

(45) Date of publication and mention of the grant of the patent:
**17.04.2019 Bulletin 2019/16**

(21) Application number: **15724591.1**

(22) Date of filing: **18.05.2015**

(51) Int Cl.:
*G06F 17/30* (2006.01)    *G06K 9/62* (2006.01)
*G06F 19/00* (2018.01)

(86) International application number:
**PCT/EP2015/060913**

(87) International publication number:
**WO 2016/184498 (24.11.2016 Gazette 2016/47)**

(54) **AUTOMATED ENTITY-RESOLUTION METHODS AND SYSTEMS**

VERFAHREN UND SYSTEME FÜR AUTOMATISIERTE ENTITÄTSAUFLÖSUNG

PROCÉDÉS ET SYSTÈMES DE RÉSOLUTION D'ENTITÉ AUTOMATISÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.01.2018 Bulletin 2018/02**

(73) Proprietor: **Hewlett Packard Enterprise Development LP**
**Houston, TX 77070 (US)**

(72) Inventors:
• **FORMOSO, Saul**
 **Stoke Gifford**
 **Bristol BS34 8QZ (GB)**
• **VAQUERO GONZALEZ, Luis Miguel**
 **Stoke Gifford**
 **Bristol BS34 8QZ (GB)**
• **WILCOCK, Lawrence**
 **Stoke Gifford**
 **Bristol BS34 8QZ (GB)**

(74) Representative: **HGF Limited**
**Fountain Precinct**
**Balm Green**
**Sheffield S1 2JA (GB)**

(56) References cited:
**US-A- 6 134 541          US-A1- 2004 107 205**
**US-A1- 2014 156 606**

• **AHMED K ELMAGARMID ET AL: "Duplicate Record Detection: A Survey", IEEE TRANSACTIONS ON KNOWLEDGE AND DATA ENGINEERING, IEEE SERVICE CENTER, LOS ALAMITOS, CA, US, vol. 19o, no. 1, 1 January 2007 (2007-01-01), pages 1-16, XP011144331, ISSN: 1041-4347, DOI: 10.1109/TKDE.2007.250581**
• **INDRAJIT BHATTACHARYA ET AL: "Collective entity resolution in relational data", ACM TRANSACTIONS ON KNOWLEDGE DISCOVERY FROM DATA (TKDD), vol. 1, no. 1, 1 March 2007 (2007-03-01), pages 5-es, XP055246003, US ISSN: 1556-4681, DOI: 10.1145/1217299.1217304**

## Description

### BACKGROUND

There are many contexts and applications (use cases) in which it may be desired to perform entity resolution (also called entity reconciliation), that is, a process to identify, within a data set, the data records that concern or relate to the same entity.

In principle entity resolution could be performed manually. However there are contexts in which it may be desired to automate the process of entity resolution, for example, in a case where manual operation would be impractical in view of the volume and complexity of the data to be processed, and/or in view of the time that would be required for manual processing.

US6134541 discloses a multidimensional data indexing technique that generates compact indexes such that most or all of the index can reside in main memory at any time.

The article "Collective Entity Resolution in Relational Data" by Bhattacharya et al. provides an entity resolution method based on a relational clustering algorithm that uses both attribute and relational information.

### Summary

The invention is defined in independent claims 1, 3 and 5. Optional features are defined in the dependent claims 2, 4 and 6.

### BRIEF DESCRIPTION OF THE DRAWINGS

Figure 1 is a flow diagram illustrating an example automated entity-resolution method according to the present disclosure;

Figure 2 is a flow diagram illustrating an example of use of the classifier set-up in the example method of Fig.1 to apply entity-resolution on updated data records;

Figures 3A and 3B are flow diagrams illustrating different example methods of determining regions in a multidimensional space, that may be implemented as part of the example automated entity-resolution method illustrated in Fig.1;

Figure 4 is a flow diagram illustrating an example method of determining a range associated with transformed cluster data in a multidimensional space, that may be implemented as part of the example method illustrated in Fig.3B;

Figure 5 is a flow diagram illustrating an example technique to select a dimension-reducing method that may be implemented as part of the example automated entity-resolution method illustrated in Fig.1;

Figure 6 is a flow diagram illustrating another example technique to select a dimension-reducing method that may be implemented as part of the example automated entity-resolution method illustrated in Fig.1;

Figure 7 is a flow diagram illustrating an example method that may trigger re-definition of classification regions in conjunction with the example method of Fig.1;

Figure 8A is a flow diagram illustrating an example method that may trigger a virtual merge of classification regions in conjunction with the example method of Fig.1;

Figure 8B is a flow diagram illustrating an example method that may trigger a reassignment of border portions of a classification region in conjunction with the example method of Fig.1;

Figure 8C is a flow diagram illustrating an example method that may trigger creation of a new region within an existing indexing region in conjunction with the example method of Fig.1;

Figure 9 is a diagram illustrating an example computer-readable medium storing machine-readable instructions that, when the instructions are executed by a processor, may implement automated entity-resolution methods according to examples of the present disclosure;

Figure 10 is a diagram representing an example of a system according to the present disclosure;

Figure 11 is a flow diagram illustrating an example automated entity-resolution method according to the present disclosure, in which virtual merges of classification regions may be implemented; and

Figures 12A to 12H are a series of diagrams to illustrate aspects of certain data-record processing in some example automated entity-resolution methods according to the present disclosure.

### DETAILED DESCRIPTION

There are numerous contexts and applications in which it may be desired to perform entity resolution.

For example, a customer service database may, because of spelling errors or other reasons, include near-duplicate data records which relate to the same customer. Each data record may have several fields (e.g. for the customer's name, address, their product IDs, etc.), and comparison of the data in these fields may make it possible to find the duplicated data records so that, for example, they can be merged into a single entry. Another example context relates to the merging of different databases - for example, when a customer service database is merged with a marketing database. In this example context, entity resolution methods may be used to identify the data records from the different databases which relate to the same customer, so that the merged database will combine out of the two databases the data that relates to this one customer.

In another example context, it may be desired to analyse a Twitter® feed in order to identify tweets that relate to the same concept, news story, event, person or place. Comparison of sets of words in different tweets may make it possible to identify tweets that relate to the same subject.

Many more contexts exist where it may be desired to perform entity resolution. However, it will be understood already from the examples above that:

- entity resolution methods tends to be used on data sets that contain data records that have a plurality of attributes,
- entity resolution methods are applied to data sets that comprise data records that concern, or relate to, subjects that may be different from or the same as one another: the expression "subject" being used in this document to include "subjects" in the sense of "concepts" as well as "subjects" in the sense of items having a material nature in the real world, e.g. individuals (people, animals), objects, places, events and so on (in this document, the word "entity" is used as a generic term to cover all types of subject to which a data record relates),
- entity resolution methods generally identify data records that relate to the same entity based on an analysis of the attributes of the data records, and
- the nature of the "data record" varies according to the context in which the entity resolution is performed. Although, the expression "data record" is frequently used to denote an entry in a database, in this document the expression is used by extension to denote, other items of multidimensional data which are the object of entity resolution methods (in addition to denoting database entries). Thus, the expression "data record" covers the items in the following non-exhaustive list: an entry in a database; the content of a text message (SMS), email, tweet, forum post, Facebook post, webpage, text file, telephone conversation, audio file, etc.; meta-data associated with programmes in an electronic programme guide and, more generally, digital content and/or a set of tags (meta-data) tagging digital content; and so on.

It may be desired to automate entity resolution, for example to speed up the process, and in situations where the data to be processed is complex and/or voluminous. It is often desired to automate the entity-resolution process in "big data" applications - i.e. applications that involve extremely large data sets that may, upon analysis, reveal associations, patterns and/or trends.

Automated entity resolution methods, i.e. which run with little or no human intervention, tend to be implemented by data processing apparatus comprising a processor executing instructions. In general, automated entity resolution techniques apply a metric for assessing the similarity between data records in a data set, based on the attributes of the data records. The nature of the metric that is applied (e.g. the computation(s), the order of the steps, the attributes taken into account, and so on) generally depends on the context.

Automated entity-resolution methods may group data records into clusters based on the similarity between them. In these cases the aim of the metric may be to assign to different clusters the data records that relate to different entities and to assign data records to the same cluster if they relate to the same entity.

Automated entity-resolution techniques differ in terms of their technical properties, for example: in terms of the computing resources they require (for instance: processing power, memory space, etc.), the time they take to deliver results, the facilities they offer, and so on.

Certain automated entity-resolution methods take a batch of data records and compare them pairwise to identify the data records that relate to the same entity. For a batch containing N data records this process involves making $N \times (N-1)/2$ comparisons between data records, and each comparison typically involves comparing some or all of the attributes of a pair of data records.

A technical challenge may exist when devising an automated entity-resolution method or system, as the required computing resources and/or time required to identify data records relating to the same entities may be undesirably great, for example in an application where it is desired to provide interactivity for a human user.

The following description presents some examples of automated entity-resolution methods and systems according to the present disclosure. Examples disclosed herein may provide technical solutions to the above technical challenges. A non-transitory computer-readable medium with machine-readable instructions stored thereon may be used to implement example automated entity-resolution methods according to the present disclosure by arranging for a processor to execute the instructions stored on the medium. An example non-transitory computer-readable medium may have machine-readable instructions stored thereon that, when executed by a processor:

obtain a group of data records of a data-set;
assess similarity between data records in the group, based on a number N of plural attributes of said data records;
identify clusters of similar data records in the group based on the assessed similarity;
determine, in a multidimensional space having a number D of dimensions less than the number N, respective regions corresponding to different clusters determined by the identifying, wherein a selected dimensionality-reduction method transforms data records into said multidimensional space; and
set up a classifier to identify correspondences between entities and updated data records compared to said group, based on the regions in said multidimensional space that contain said updated data records after transformation thereof according to the selected dimensionality-reduction method.

An example automated entity-resolution system comprises:

a classifier module to identify the correspondence between different entities and data records of a data set, said data records having plural attributes, wherein the classifier module stores definitions of respective regions in a multidimensional space;
an updating module to supply update data to add, modify or delete data records of the data-set; and
a data-transformation module to transform updated data records into said multidimensional space by application of a selected dimensionality-reduction method to plural attributes of said updated data records;
wherein the classifier module comprises:

a region-identification module to determine the respective locations of the transformed updated data records in said multidimensional space, and to determine which of said regions contain(s) the respective locations, and
an entity identification module to determine the correspondence between entities and updated data records based on: the region which contains the location of the transformed updated data record, and on an assignment of entities to the regions in the multidimensional space.

By setting up a classifier to identify correspondences between updated data records and entities it may be possible to perform entity resolution based on the definitions of the regions in the multidimensional space and on knowledge of the dimensionality-reduction method. This may obviate the need to store all the data of the previously-processed data records and, thus, reduce the amount of memory space needed for implementation. Further, this may provide a rapid detection of data records that relate to the same entity. Yet further, this technique may enable a facility to respond to queries of the type "to which entity does updated data record X relate?".

Another example non-transitory computer-readable medium may have machine-readable instructions stored thereon that, when executed by a processor:

obtain update data of a dynamically-updating data set, the update data defining at least one change selected in the group consisting of: addition of a new data record to the data-set, modification of a data record in the data-set, and deletion of a data record in the data-set;
map updated data records of the data-set into different regions of a multi-dimensional space based on attributes of said updated data records,
identify correspondences between updated data records and entities based on the regions in said multidimensional space that contain the mapped updated data records,

evaluate, for each region, the number of updated data records mapped into said region but proximate a boundary with an adjacent region, and
in a case in which the counting indicates that a specified quantity of mapped data records are proximate the boundary between a pair of adjacent regions, perform a virtual merge of said pair of regions so that updated data records mapped into either of the pair of regions is classified as corresponding to the same entity but the boundaries of the adjacent regions are unchanged and separate statistics are still maintained on the quantity of updated data records mapped into each of the adjacent regions.

By mapping updated data records, based on their attributes, into a multidimensional space having regions that are used to identify correspondences between data records and entities, counting numbers of updated data records whose mapped data is proximate the boundary between a pair of adjacent regions, and performing a virtual merge of the adjacent regions when a specified quantity is counted, accurate correspondences may be identified between data records and entities without unduly increasing the processing required to adapt the region definitions in the multidimensional space.

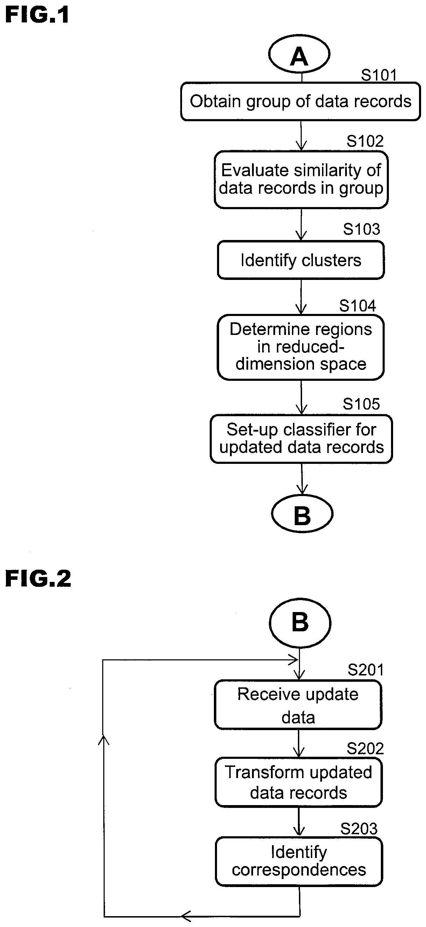

An example automated entity-resolution method is illustrated by Fig.1. The discussion of Fig.1 presented below will consider an example data-set in which each data record has a number, c, of attributes, such that any data record may be considered to be a vector S in a multidimensional space that has c dimensions, i.e.

$$\vec{S} = S1xS2xS3....xSc$$

In the example method of Fig.1, a group of data records in the targeted data-set is obtained (S101), for example by reading out of a memory, by receipt from a data buffer, by receipt from an external device, and so on. Then the data records in the group are processed to assess their similarity to each other (S102). Numerous metrics for assessing similarity are known and the methods according to this disclosure are not limited having regard to the particular method that is used for assessing similarity: one of the known methods may be used, and it is expected that similarity-evaluation techniques that may be developed in the future would also be useable. However, it is appropriate to note that the metric which evaluates similarity makes its evaluation taking into account a number N of attributes of the compared data records (i.e. considering the values of the N attributes for each of the data records in the group). The number N of attributes taken into account for assessing similarity may include all the available attributes of the data records under consideration (i.e. the c attributes mentioned above), or a lesser number of attributes, and the number and selection of attributes that are used may depend on the use case. The system designer may pre-specify the N attributes to be used for assessing similarity. In many real-world contexts the number N will be relatively large, especially in applications that aim at perform entity-resolution as part of a process for merging data from plural data sources (e.g. plural databases) .

In the example entity-resolution method illustrated in Fig.1, clusters of similar data records are identified within the considered group (S103). Various clustering techniques are known and the methods according to the present disclosure are not particularly limited having regard to which clustering method is used one of the known methods may be used, and it is expected that clustering techniques that may be developed in the future would also be useable.

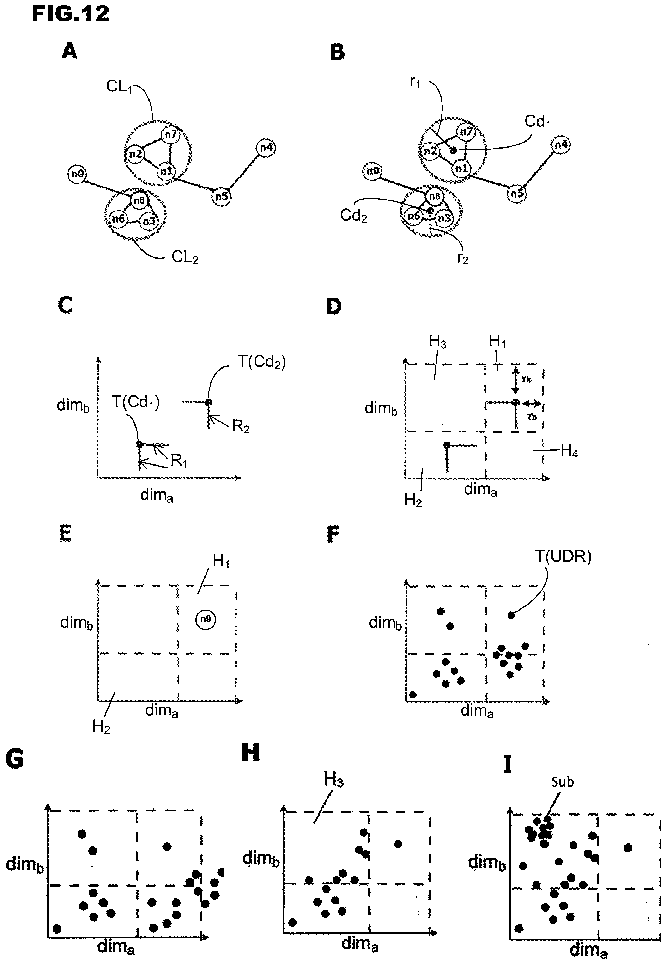

For illustrative purposes, Fig.12A shows a diagram to illustrate a possible result of clustering performed on a set of data records n0 to n8. Fig.12A is a two-dimensional drawing illustrating clustering but it should be mentioned that, in practice, the clustering is liable to take place in a hyper-dimensional space that cannot be represented in three dimensions. In the example illustrated in Fig.12A, a first cluster $CL_1$ has been identified containing the three data records n1, n2 and n7 (which have been assessed as being similar to one another), and a second cluster $CL_2$ has been identified containing the three data records n3, n6 and n8 (which, likewise, have been assessed to be similar to one another). Each of the data records n0, n4 and n5 is treated as a cluster in its own right.

Incidentally, in various known entity-resolution methods a blocking operation is performed prior to performing clustering of data records. Blocking comprises dividing an initial set of data records into blocks such that data records in different blocks are known to definitely correspond to different entities. Data records assigned to the same block might relate to the same entity but there is still an element of uncertainty in this regard. Although the example method described with respect to Fig.1 does not include a blocking operation it should be understood that the method may be extended to include a preliminary blocking operation performed prior to point A in Fig.1.

In various contexts it is desired to perform entity-resolution on a data set that is not static, that is, on a data set that is subject to updating events that may introduce new data records and/or modify or delete existing ones. In a case in which automated entity-resolution techniques are applied to a data set that is dynamic in this way, it may be desired to revise the assessment of which data records relate to the same entities, and for the revision to take into account the content of the updating events - not only to determine whether newly-added data records relate to the same entities as existing data records, but also to improve or correct determinations made previously.

Incidentally, the expression "updating" (and related terms) as used in this document does not imply or infer that the update event must necessarily be an event that provides new/changed data to a data set: to the contrary, the update event may comprise taking into account the next chunk of data that is already present in a data set (i.e. the entity-resolution process may operate on different portions of a given data-set in a progressive manner).

A naïve approach to entity resolution in a context where the data set experiences updating events would be to re-run the similarity-evaluation metric on the updated batch of samples, i.e. re-apply the entity resolution method to the batch of previous samples as updated by the updating event(s). Such an approach requires ever-increasing computational resources as the number of data records in the updated batch increases, and the entity-resolution process could take an excessively long time.

Other automated techniques have been proposed, including some "incremental" approaches which do not re-compute everything from scratch upon occurrence of an updating event. In general, incremental entity resolution techniques only perform computations relating to the changes that result from an updating event. Thus, for example, an entity-resolution method that evaluates similarity between data records and clusters similar data records might seek to make incremental adjustments to the clusters in response to an update event. In general, known incremental approaches store the attribute data of the data records considered to date. Thus, although the amount of computation may be limited with such incremental methods, they still require ever-increasing amounts of memory space to store details of the data records.

Some so-called "streaming" methods have been proposed, in which entity resolution is performed only over a window containing the most recent data records (e.g. only the data records received during the last X minutes, only the last Y data records, etc.). Although such methods do not entail the use of an endlessly-expanding memory space, they may still require use of a substantial amount of memory to store attribute data of the data records in the current window, especially in a case in which the data records have a large number of attributes (a large number of dimensions).

The example entity-resolution method illustrated in Fig.1 may perform entity resolution in a context where the data set in question is not static, including contexts involving a "streaming" data set. However, the example method of Fig.1 does not require storage of all the attribute data of the considered data records (neither of all the data records considered to date, nor all the data records in a window).

According to the example method illustrated in Fig.1, after clusters of similar data records have been identified (by analysis of an initially-considered group of data records), the cluster data is processed using a dimensionality-reduction technique so as to identify - in a multidimensional space that has fewer dimensions than the number of attributes that were taken into account to assess similarity and identify the clusters - respective regions that corresponds to the different clusters and which are well-separated from each other (S104). So, for instance, if N attributes of the data records were used to assess similarity then the multidimensional space in which the regions are identified may have D dimensions, where D<N.

Dimensionality-reduction techniques suitable to process the cluster data to identify appropriate regions in a multidimensional space of reduced dimensions have been proposed in the field of statistical analysis and may be applied in example entity-resolution methods according to the present disclosure. Suitable dimensionality-reduction techniques include, but are not limited to: principal components analysis (PCA), discriminant factorial analysis (DFA), hidden Markov model (HMM) techniques, state-space model techniques, mixtures of Gaussians, as well as variants and extensions of the enumerated techniques.

The reduced set of dimensions determined using the selected dimensionality-reduction technique may or may not have real-world meaning. Thus, although the dimensions that define the multidimensional space of reduced dimensions may, in an example, correspond to a subset of the attributes of the considered data records, some or all of the dimensions could alternatively or additionally correspond to a transformation and/or combination of attributes.

Fig.12C provides a simplified example of a multidimensional space having reduced dimensions (i.e. dimensions labelled as $dim_a$ and $dim_b$). Fig.12D illustrates the reduced-dimension space of Fig.12C with an example set of region boundaries marked using dashed lines. In the example of Fig.12D, the multidimensional space of reduced dimensions has been divided to define region boundaries set so that transformed data relating to cluster $CL_1$ is in a different region (labelled H1) from transformed data relating to cluster $CL_2$ (which is in the region labelled H2). However, the dividing surfaces which define the regions H1 and H2 also define additional regions H3 and H4 in the multidimensional space $\{dim_a, dim_b\}$ discussed below.

In the example method illustrated by Fig.1, a classifier is set up (S105) so that it can identify correspondences between entities and data records based on the regions that have been defined in the multidimensional space of reduced dimensions. In particular, the classifier is set up to identify a correspondence between a data record and a particular entity if, responsive the data record being transformed into the multidimensional space of reduced dimensions, the transformed data is located within a region associated to the entity in question. The nature and/or identity of the entity in question is not necessarily known a priori. A finding that two data records are transformed into the same region may be interpreted as a finding that the two data records relate to the same entity. This may be sufficient for entity-resolution purposes in certain contexts.

The classifier may be set up to implement a hyper-dimensional indexing process, with different index values being assigned to different regions in the multidimensional space of reduced-dimensions. In order to be able to implement this indexing process it may not be necessary to permanently store the attribute data of the data records that contributed to definition of the indexing regions; what may be necessary is to know the dimensionality-reduction transformation and the definitions of the regions. Thus, it is permissible to discard the attribute data of the data records that served in the process defining the indexing regions, and such discarding may enable a reduction in the memory space required for performance of the entity-resolution process.

The classifier may be built in different ways. For example, the classifier may be built of a computation module that uses a set of linear classifier functions to determine the region in which a data record's transformed data lies. Another approach comprises using a neural network and/or decision trees to determine the region containing a data record's transformed data. Moreover, other approaches may be used. In examples where the reduced-dimension space still has many dimensions it may be efficient to use a neural network implementation.

In the example method illustrated by Fig.1, the set-up of the classifier in S105 may be based on clustering, dimensionality-reduction and region-identification processes all of which are unsupervised, that is, do not involve a priori knowledge of which data record relates to which subject or which data records relate to a common subject. However, in other example methods the set-up of the classifier may be semi-supervised, that is, the set-up of the classifier may take place in the context of an expert system in which a human intervenes at one, two, or more than two "checkpoints", to validate prior decisions before the processing of the entire data set is performed. In a case where large data sets are being processed it may be beneficial to make use of unsupervised methods for assessing similarity between the data records of the group and for clustering.

In the example method illustrated by Fig.1, the set-up of the classifier in S105 is based on the clustering, dimensionality-reduction and region-identification that were performed based on the group of data records obtained at S101. The classifier is thus ready to classify updated data records resulting from the update of the data set relative to the original group of data obtained at S101.

Fig.2 illustrates an example of use of a classifier that has been set-up by the example method of Fig.1 to apply entity-resolution taking into account updated data records. The reference B indicates that the example method of Fig.2 may follow on from step S105 in Fig.1.

In the example method of Fig.2, update data is received (S201). The update data may take different forms and it may comprise: data which defines a data record that has not yet been considered in the entity-resolution process, and/or data which defines a change or deletion of a data record that has already been considered in the entity-resolution process. The update data may be received in the same manner, or a different manner, from the group of data records used to set up the classifier.

In the example method of Fig.2, updated data records are transformed into the multidimensional space in which the classification regions have been defined (S202). The transformation is performed according to the dimensionality-reduction method that was used in the determination of the indexing regions. The classifier then identifies the correspondence between the updated data records and entities based on the respective region(s), in the multidimensional space, which contain(s) the transformed data.

For the purposes of illustration, Fig.12E illustrates the case of an updated data record n9 which is additional to the group of data records n0 to n8 that were used in the process of defining regions in the reduced-dimension space $\{dim_a, dim_b\}$ in Fig.12D. According to this example, in a case in which the attributes of data record n9 are transformed according to the same dimensionality-reduction method as that which gave rise to the indexing regions, the transformed updated record data is located in region H1 shown at the top right of Fig.12E. Accordingly, updated data record n9 may be considered to correspond to an entity associated with region H1 and other data records whose data is transformed into region H1 may be considered to relate to the same entity as n9.

Various technical benefits may be obtained from using the example automated entity-resolution method of Fig.1. For example, it is possible to employ this example method to perform membership searches, that is, to provide a response to queries of the kind "what entity/group does data record x belong to?" (The answer could be expressed in terms of an index value assigned to the region containing the transformed data record x). This facility is something which is not usually possible with other automated methods: other automated entity-resolution methods produce as their work product just a group of indexed data records, without any mechanism for processing membership queries. Further, the computational "cost" involved in performing a membership search (i.e. the time taken and/or the required computational resources) is relatively low in a case where the automated entity-resolution method of Fig.1 is used.

The example automated entity-resolution method of Fig.1 may enable performance of entity-resolution in a streaming manner without a requirement for a priori knowledge of the number of duplicates/equivalent data records. This is different from known streaming techniques that are usually based on use of k-means or k-meioids, and provides the technical benefit that it increases the range of contexts, involving streaming data, in which entity-resolution may be performed in an automated manner.

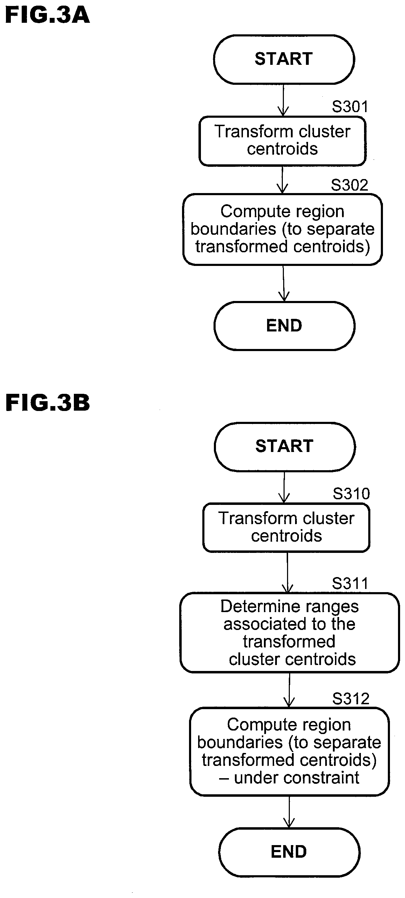

Fig.3A illustrates an example method that may be used to implement the determination of regions indicated at S104 of Fig.1.

According to the example method of Fig.3A, a selected dimensionality-reduction method is applied (S301) which transforms the centroids of the identified clusters into a multidimensional space of reduced dimensions (i.e. reduced compared to the number of dimensions used for similarity assessment and identification of the clusters). Fig.12B reproduces the clustering example of Fig.12A but, in addition, shows the respective centroids, Cd1, Cd2, of the clusters CL1 and CL2. In the example multidimensional space $\{dim_a, dim_b\}$ illustrated in Fig.12C T(Cd1) and T(Cd2) label positions corresponding to the locations, in the reduced-dimension space, of the transformed data of the centroids of clusters $CL_1$ and $CL_2$, that is the locations of the centroids of the clusters CL1 and CL2 after they have been mapped into the new space by the selected dimensionality-reduction method.

According to the example method of Fig.3A, there is a computation of boundaries to define regions, within the multidimensional space, that correspond to the different clusters and the region boundaries are defined so that each region contains only one cluster centroid It can be seen from Fig.12D that the region boundaries have been defined in a manner such that the transformed centroid Cd1 of cluster $CL_1$ is in a different region (H1) from the region (H2) containing the transformed centroid Cd2 of the cluster $CL_2$.

Fig.3B illustrates another example method that may be used to implement the determination of regions indicated at S104 of Fig.1.

According to the example method of Fig.3B, a selected dimensionality-reduction method is applied (S310) to transform the centroids of the identified clusters into a multidimensional space of reduced dimensions, as in S301 of the example method of Fig.3A. However, the example method of Fig.3B includes an additional process (S311) of determining ranges associated to the transformed cluster centroids. The range associated to a transformed centroid is a measure of the spread of the cluster in the reduced-dimension space.

There are various methods available for determining the associated range. Fig.12 illustrates an example using a determination method in which the size of the radius r of a cluster is used to determine the range R associated with the transformed cluster centroid in the reduced-dimension space. That is, a point located at the circumference of the cluster (a distance r away from the centroid) is transformed into the reduced-dimension space and is mapped to a location that is a distance R away from the transformed centroid.

As shown in Fig.12B cluster $CL_1$ has an assigned radius $r_1$ and Fig.12C shows that this produces a range $R_1$ associated with the transformed centroid T(Cd1). Fig.12 relates to a case where the dimensionality-reduction method is such that points which are close to or distant from one another in the space containing the original clusters are still close to or distant from one another in the reduced-dimension space $\{dim_a, dim_b\}$.

This proportionality of the original and transformed spaces may exist, for example, in cases where PCA is used and the reduced-dimension space has a relatively small number of dimensions. Further, Fig.12 relates to a case in which the original clusters are circular. Use of the cluster radius to define the range associated with the cluster in the reduced-dimension space is not always the optimal approach, especially in examples that use other dimensionality-reduction methods and/or in examples in which the original clusters are not circular.

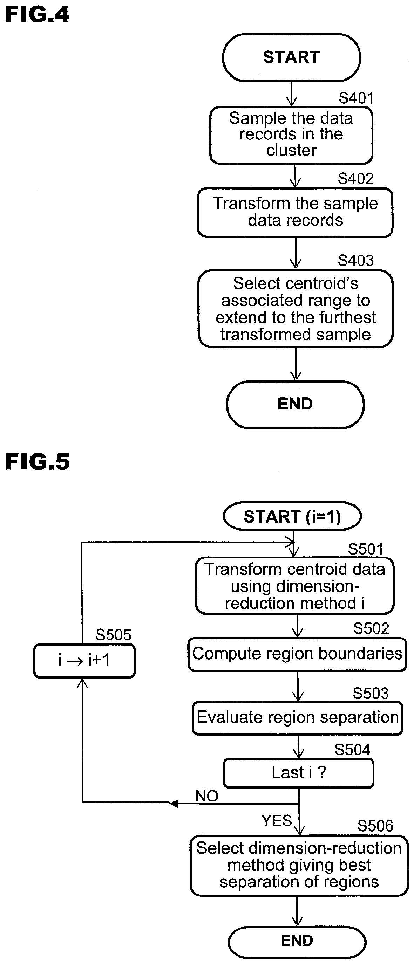

Fig.4 illustrates a different example method for determining the range associated to a cluster in the reduced-dimension space. The example method of Fig.4 comprises: taking a sample of data records within a given cluster (S401), transforming the data records of the sample into the reduced-dimension space (S402), and then determining, as the range associated with the cluster in the reduced-dimension space, a distance extending from the transformed cluster centroid to the furthest of the transformed data records of the sample (S403). In certain example implementations of the Fig.4 method, the sampling of data records in the given cluster takes a random sample of records within the cluster. The randomness increases the probability that the sampled records are representative and hence indicate accurate boundaries for the cluster.

The method of Fig.4 provides the benefit that it increases the independence of the range-setting process relative to the dimensionality-reduction method. That is, in examples in which this method is used to determine the range associated with a cluster in the reduced-dimension space, an appropriate value of the range can be set even in cases in which the dimensionality-reduction method does not assure proportionality between distances in the original space and distances in the reduced-dimension space. This method also provides the benefit that it enables an appropriate value of the range to be set even in cases in which the original clusters are not circular.

According to the example method of Fig.3B, there is a computation (S312) of boundaries to define regions, within the multidimensional space, that correspond to the different clusters. However, in this example method the region boundaries are defined not only so that each region contains only one cluster centroid but also with a constraint that - to the extent possible - the region assigned to a given cluster should extend to include the transformed centroid of the cluster and also points spaced from the transformed cluster centroid by a distance equal to or less than the range associated with that transformed centroid.

Fig.12D illustrates the above-mentioned approach by showing that region boundaries are set in a manner which includes in the same region a given transformed centroid and its associated range (the associated ranges are represented using lines extending from the transformed centroids).

Fig.12D illustrates an additional constraint that may be applied during the setting of region boundaries. This additional constraint holds on the outermost boundaries of the outermost regions in the reduced-dimension space (whose constituent points take the most extreme values of the dimensions defining the reduced-dimension space). According to this additional constraint, the outermost boundaries of the outermost regions in the reduced-dimension space may be set with a certain tolerance (indicated by Th in Fig.12D) to give room to accommodate outliers for the cluster in question (for instance those beyond the 95th percentile). In certain implementations the size of the tolerance level Th may be configurable by the user.

As mentioned above there are various different methods available for performing dimensionality-reduction. Certain example methods according to the present disclosure may perform a machine-learning technique to select a dimensionality-reduction method to apply during implementation of an entity-resolution method such as that of Fig.1. Fig.5 illustrates an example machine-learning technique that may be employed.

According to the example machine-learning technique illustrated by Fig.5, a group of i dimensionality-reduction techniques are candidates for selection. In this example, a first one of the dimensionality-reduction methods is used to transform centroid data into a reduced-dimension space (S501) and then region boundaries are computed based on the centroid data transformed according to this first dimensionality-reduction method (S502). An assessment is then made of "how good" the regions are that are produced using this first dimensionality-reduction method. This assessment may be made by evaluating (S503) how well-separated the different regions are from one another (because it may be considered that it is easier to discriminate between different entities if they correspond to regions which are widely-spaced apart from one another in the reduced-dimension space).

A variety of techniques are available for evaluating the separation between regions. One technique comprises quantifying the number of boundary points of a cluster in the untransformed space that fall into a different clustering in the transformed space. A high value for the number of boundary points which have changed cluster after the dimension-reduction indicates that the transformed clusters are too close together (excessive overlap). In a similar way, a low value for the number of boundary points which have changed cluster after the dimension-reduction indicates that the transformed clusters are well separated. Thus, the quantified number of boundary points may be used as an indicator of how well separated the defined regions are in the reduced-dimension space. Other techniques may be used to evaluate the separation between regions and to yield a parameter value quantifying the separation.

After the region separation obtained using the first dimensionality-reduction method has been evaluated, a check may be made (S504) as to whether or not there is another candidate dimensionality-reduction method and, if there is another candidate method then a parameter i labelling the methods may be incremented before processes S501 to S504 are repeated for the next candidate dimensionality-reduction method. Responsive to region boundaries having been computed using the last of the candidate dimensionality-reduction methods, a selection is made of the candidate method which yielded the most well-separated regions (S506).

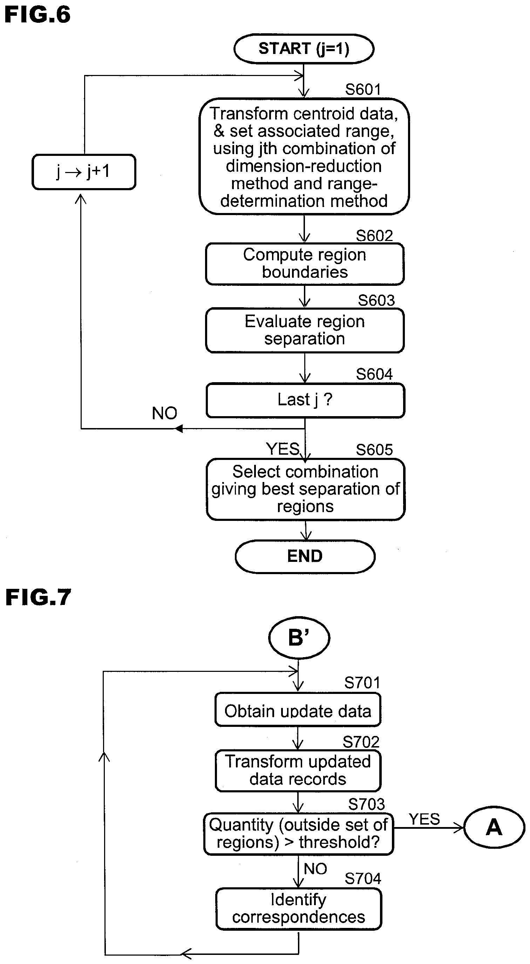

As mentioned above there are different methods available for determining the ranges to be associated to clusters in the reduced-dimension space. Certain example methods according to the present disclosure may perform a machine-learning technique to select a combination of a dimensionality-reduction method (to apply during implementation of an entity-resolution method such as that of Fig.1), and a method for determining the associated range for a cluster in the reduced-dimension space. Fig.6 illustrates an example machine-learning technique that may be employed.

According to the example machine-learning technique illustrated by Fig.6, there is a group of j combinations of dimensionality-reduction techniques and range-determining methods that are candidates for selection. For instance, the system designer may specify in advance that there are p different dimensionality-reduction techniques that should be considered and that each of these may be combined with any one of q different approaches for determining the ranges to be associated to clusters: yielding a total number j = p x q of candidate combinations. As another example, the system designer may specify a set of specific combinations of dimensionality-reduction techniques and range-determining methods as the candidate combinations that are to be tested.

In this example, a first one of the candidate combinations is used to transform centroid data into a reduced-dimension space and set the associated range (S601) and then region boundaries are computed (S602). An assessment is then made of "how good" the regions are that are produced using this first dimensionality-reduction method. This assessment may be made by evaluating (S603) how well-separated the different regions are from one another.

After the region separation obtained using the first candidate combination of methods has been evaluated, a check may be made (S604) as to whether or not there is another candidate combination and, if there is another candidate combination then processes S601 to S604 are repeated for the next candidate combination. Responsive to region boundaries being computed using the last of the candidate combinations of methods then a selection is made of the candidate combination which yielded the most well-separated regions (S605).

The description above relating to Fig.2 presented certain processing that may be performed, in example entity-resolution methods according to the present disclosure, in cases in which the targeted data set is subject to being updated. A description will now be given of Figs.

7, 8A and 8B, which illustrate certain example methods in which update data relating to a targeted data set may affect an indexing framework set up using a method such as that of Fig.1.

As has been mentioned above, in cases in which entity-resolution is performed using the example automated method according to Fig.1 it may be permissible to discard the attribute data of the group of data records that serve to generate the indexing framework, and in instances in which the attribute data is discarded this reduces the memory space required to implement the method. In certain example methods according to the present disclosure the attribute data of the updated data records that are mapped into the indexing framework may likewise be discarded (further reducing the memory space required for the entity-resolution process).

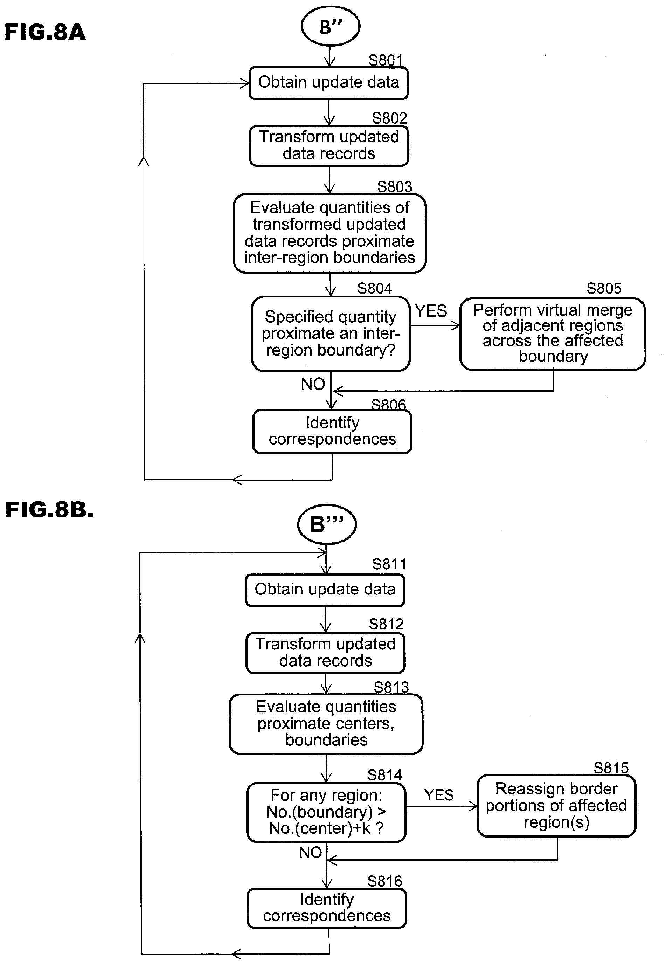

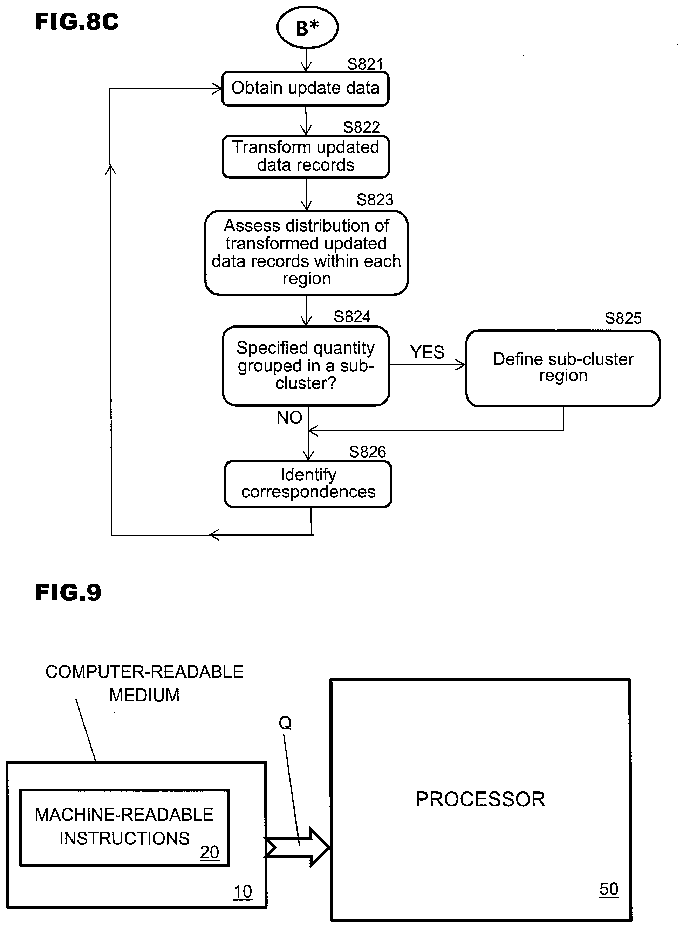

Figs.7, 8A, 8B and 8C relate to example methods in which the attribute data of the updated data records mapped into the indexing framework is discarded but statistics are maintained regarding where in the reduced-dimension space updated data records have been mapped. In some examples, as described further below, the statistical data relating to a given updated data record may be discarded in certain example implementations that take a streaming approach.

Figs.7, 8A, 8B and 8C will be described in conjunction with references to Figs.12F to Fig.12I. In Figs.12F to 12I dots such as that labelled T(UDR) represent positions in the reduced-dimension space {$dim_a$, $dim_b$} corresponding to updated data records after they have been transformed into the indexing space using the selected dimensionality-reduction method.

Fig.7 relates to an example method that detects and responds to a situation in which a significant number of the updated data records being mapped onto the indexing framework have transformed locations that are outside the outermost of the regions that have been defined for the indexing framework. A simplified example of such a situation is illustrated in Fig.12G, considering the group of dots at the extreme right-hand side.

What constitutes a "significant" number of updated data records mapped to locations falling outside the outermost regions defined for the indexing framework tends to depend on the use case. Whether or not it is significant that a given number of data records fall outside the outermost of the regions defined for the indexing region may depend on the probability that this number could arise in the case of an indexing framework whose regions accurately represent the categories present in the data set. Various techniques (including power analysis, sample size estimation, advanced techniques for confidence interval estimation, and so on) may be used to detect the situation where a particular detected number of updated data records mapped to locations falling outside the outermost regions defined for the indexing framework is "significant" in a given context/use case.

In cases in which a significant number of the mapped updated data records fall outside the indexing framework that may be a sign that the targeted data-set is evolving (In a case where the update data represents newer data than the original group of data records) or that the data records in the original group were not fully representative of the whole targeted data set (in other cases). It may be desired to provide for a recalculation of the indexing framework in such circumstances, because it may be possible that the entity-resolution process would produce results of improved accuracy using a recalculated indexing framework that is determined taking into account the updated data which (upon being transformed) fell outside the previous indexing framework.

The recalculation of the indexing framework may comprise performing afresh all the operations represented in Fig.1 using a different group of data records. This different group of data records may comprise all the data records processed to date (i.e. the original group used to generate the previous indexing framework as well as all updated data records input since that framework was generated), just updated data records input since the previous indexing framework was generated (all of the input updated data records or a sub-set), or a selection from the original group and the updated data records. Including updated data records in the group used to recalculate the indexing framework may mean that the freshly calculated indexing framework takes into account evolution in the data set.

Because the decision to recalculate the indexing framework may be taken in circumstances where the data set is evolving, it may be appropriate, as part of the recalculation process, to reassess which attributes should contribute to the definitions of the dimensions of the reduced-dimension space (because a different choice from before may produce an indexing space that better fits the properties of the data set in its current state of evolution). However, in view of the time and computational resources that are necessary to make the assessment, it may be decided to keep the existing choice of dimensions for the reduced-dimension space, and to recalculate regions within that space.

A consideration weighing against frequent reindexing is the time and computational resources it takes to recalculate the indexing framework. However, in order to be able compute a new indexing framework taking into account the updated data records that fell outside the preceding indexing framework, it is necessary to store data of the relevant "outlier" data records (and perhaps data of other updated data records obtained at a similar time) until the reindexing operation takes place. So, a long wait before performing reindexing might entail a need to store a relatively large quantity of data.

According to the example method of Fig.7, the indexing framework is recalculated (using processes as in Fig.1) responsive to a determination being made that the quantity of updated data records mapped to locations outside the set of indexing regions exceeds a threshold.

The reference B' in Fig.7 indicates that the example method of Fig.7 may follow on from step S105 in Fig.1 (in place of the example method illustrated in Fig.2). Indeed, the first processes S701 and S702 of the Fig.7 method - to obtain update data and transform updated data records into the reduced-dimension space,- may be implemented in the same way as the first processes S201 and S202 in the Fig.2 method.

However, according to the example method of Fig.7 statistics are gathered regarding the number of updated data records being mapped to locations outside the outermost of the regions in the indexing framework. A simple approach for gathering the relevant statistic is simply to maintain a running total of the number of updated data records that are mapped to such locations. However, other approaches may be used.

For instance, the count may be restarted from zero after a certain period of time, or after a certain number of updated data records has been processed. These are examples of counting within a so-called "tumbling window": the first case corresponds to a window defined in terms of a certain time period and the second case corresponds to a window defined in terms of a certain number of data records. As another example, the count may be made in a sliding window (defined in terms of a time period or in terms of a number of data records).

Different approaches may be used to determine how a time-based window may be set. The time window may relate to the time when the relevant update data is transformed into the reduced-dimension space, the time when the relevant update data was obtained, a time stamp associated with the update data, etc. The duration of the window may take into account the memory resources that are available. The approach used to set a time-based window may depend on the use case/application.

According to the example method of Fig.7, a check is made as to whether the evaluated quantity of updated data records mapped to locations outside the set of indexing regions exceeds a threshold (S703). If the threshold is exceeded then the indexing regions are recalculated but if the threshold is not exceeded indexing continues (S704) based on the existing indexing framework. The threshold level may be set sufficiently high to avoid frequent reindexing operations (in view of the time these require) but sufficiently low to avoid an undue delay between onset of change in the updating data set and adaptation of the indexing schema. In certain implementations the level of the threshold may be configurable by the user. For the purposes of illustration, consideration may be given to a use case which seeks to perform "de-duplication" in a database of patients in a national healthcare system (e.g. in the UK's National Health Service, or "NHS"), to reduce the number of duplicate or near-duplicate entries relating to the same patient. In a use case of this type it may be decided to set the threshold level that triggers recalculation of the indexing framework to a low level, e.g. 1% of the data records considered in the reduced-dimension space at the time when the threshold is applied (e.g. ignoring data records outside a current processing window).

The example method of Fig.7 may be adapted and varied in various ways. For instance, in certain implementations it may be desired to trigger a reindexing operation as soon as a single outlier is detected (i.e. a single updated data record that is mapped to a location outside the indexing regions). As another example, in some cases it may be preferred not to trigger a reindexing operation in response to detection of an outlier and, instead, the outlier may be mapped to the adjacent extreme (maximum or minimum) dimension value that corresponds to an indexing region. Just in case the "outlier" is not truly an outlier but instead is an example of a change in the data, details of the "outlier" may be kept in a buffer for processing in a future reindexing operation (to take place responsive to detection of a number of other "outliers").

In certain example implementations, for instance in some example methods where the input update data is obtained from a window manager that buffers a stream of input data items, it may be desired to postpone a reindexing operation if the window manager still holds update data waiting to be supplied (the reindexing operation may be performed when the window manager's buffer next becomes empty).

Fig.8A relates to an example method that detects and responds to a situation in which a significant number of the updated data records being mapped onto the indexing framework are proximate a particular inter-region boundary (between a pair of adjacent regions). A simplified example of such a situation is illustrated in Fig.12F, considering the group of dots straddling the boundary between region $H_1$ and region $H_4$.

What constitutes "a significant number" of updated data records mapped to locations that are proximate a particular inter-region boundary tends to depend on the use case. Various techniques (including power analysis, sample size estimation, advanced techniques for confidence interval estimation, and so on) may be used to detect the situation where a particular detected number of updated data records mapped to locations that are proximate a particular inter-region boundary is "significant" in a given context/use case.

In a case in which a significant number of the mapped updated data records are close to a particular inter-region boundary this may be a sign that the adjacent regions at either side of this boundary actually relate to the same entity. In such circumstances it might be considered desirable to remove the boundary between the adjacent regions and assign a single index to the combined regions. A somewhat different approach is taken in the example method of Fig.8A.

The reference B" in Fig.8A indicates that the example method of Fig.8A may follow on from step S105 in Fig.1 (in place of the example method illustrated in Fig.2). The first processes S801 and S802 of the Fig.8A method - to obtain update data and transform updated data records into the reduced-dimension space,- may be implemented in the same way as the first processes S201 and S202 in the Fig.2 method.

However, according to the example method of Fig.8A method statistics are gathered, for each of the indexing regions, regarding the number of updated data records being mapped to locations proximate the boundaries of this region with each of its nearest neighbours.

A simple approach for gathering the relevant statistics is simply to maintain, for each indexing region, running totals of the numbers of updated data records mapped to the different peripheral portions of this region that are adjacent to each nearest neighbour region. However, other approaches may be used. For instance, the statistics may be evaluated in respect of updated data records in a given sliding or tumbling window (defined in terms of time or in terms of a number of data records), as for the example method of Fig.7.

A finding that a large number of updated data records has been mapped to a specific peripheral portion of an indexing region adjacent to a specific other region may give a misleading impression unless some account is taken of the number of updated data records that have been mapped to other parts of the same region (e.g. to other peripheral portions, to the central portion). Accordingly, in certain implementations of the example method of Fig.8A, an additional evaluation is made of the number of updated data records mapped to the central portion of each region. (This could be an explicit count of the numbers mapped to the central region, a computed value determined by comparing the total number of records mapped to this region and the numbers of records mapped to the peripheral portions, and/or other type of evaluation).

According to the example method of Fig.8A, an evaluation is made of the quantities (number or proportion) of transformed updated data records that are proximate an inter-region boundary (S803). This evaluation may consider each region separately or it may consider points located in the regions on both sides of a given boundary. If it is determined (S804) that a specified quantity of transformed updated data records are proximate a given inter-region boundary then, according to the example method of Fig.8A, a "virtual merge" (S805) may be performed between the adjacent regions on either side of the boundary in question (see below). The specified quantity may be expressed in terms of a threshold number or proportion, and the facility may be provided for a user to configure the threshold. If the result of the determination S804 is negative then the indexing continues (S806) based on the existing indexing framework without a virtual merge.

An actual merge between two adjacent regions Rv and Rw would comprise redefining the region boundaries and replacing the two adjacent regions by a single new larger region Rz that is the union of the two previous adjacent regions (Rz = Rv ∪ Rw). After an actual merge updated data records whose transformed data lies anywhere within the new region Rz would be classified as relating to the same subject. Statistics gathered in relation to the location of transformed data within regions would be evaluated for the new larger region Rz as a whole.

In contrast, in the present example a "virtual merge" between two adjacent regions R'v and R'w does not actually replace the two adjacent regions with one new merged region. To the contrary, statistics gathered in relation to the location of transformed data within regions is still evaluated individually for the adjacent regions R'v and R'w that are the object of the virtual merge. However, updated data records whose transformed data falls into either of the two regions R'v and R'w is assigned to the same entity.

Thus, according to the example method of Fig.8A, a virtual merge between a pair of adjacent indexing regions does not remove the boundary between the adjacent regions. To the contrary, the two adjacent regions are still maintained as individual units, and for updated data records that are mapped into the indexing space an account is taken of which of the adjacent regions contains each item of mapped data (for example, the counts of data records mapped into central and peripheral portions of regions is still maintained individually for the two adjacent regions). However, while the adjacent regions are subject to the virtual merge, updated data records classified in the two different regions are considered to correspond to the same entity (and are reported as such in response to membership queries).

Fig.8B relates to an example method that detects and responds to a situation in which a significant number or proportion of the updated data records being mapped into a particular indexing region are spread around the periphery of the region (rather than having a concentration near one particular nearest-neighbour as was the case for Fig.8A). A simplified example of such a situation is illustrated in Fig.12H, considering the distribution of dots around the edges of the region $H_3$. This kind of distribution tends to suggest that the region in question does not itself correspond to a distinctive entity but, on the contrary, the updated data records that have been mapped into this region actually relate to entities that correspond to the surrounding regions.

What constitutes a "significant" number or proportion of the updated data records being spread around the periphery of a particular indexing region tends to depend on the use case. Various techniques (including power analysis, sample size estimation, advanced techniques for confidence interval estimation, and so on) may be used to detect the situation where a particular detected number/proportion of updated data records spread around the periphery of a particular indexing region is "significant" in a given context/use case.

The reference B'" in Fig.8B indicates that the example method of Fig.8B may follow on from step S105 in Fig.1 (in place of the example method illustrated in Fig.2). The first processes S811 and S812 of the Fig.8B method -to obtain update data and transform updated data records into the reduced-dimension space - may be implemented in the same way as the first processes S801 and S802 in the Fig.8A method.

According to the example method of Fig.8B method, statistics are gathered, for each of the indexing regions, regarding the number of updated data records being mapped to locations proximate the boundaries of this region with each of its nearest neighbours and the number being mapped to the central portion of the region (S813).

Once again, the relevant statistics may be maintained by keeping running totals of the relevant parameters for each indexing region, or by other methods (e.g. evaluating the statistics in respect of updated data records in a given sliding or tumbling window defined in terms of time or in terms of a number of data records).

According to the example method of Fig.8B, an evaluation is made of whether or not the number of transformed updated data records that are proximate the boundaries of the region exceeds, by a certain amount k, the number proximate the center (S814). If the result of the S814 determination is positive then, according to the example method of Fig.8B, the peripheral portions of the affected region are reassigned to the relevant nearest neighbour regions. This reassignment changes the boundaries of the regions and redistributes updated data records that had previously been mapped into the affected region to the relevant nearest one of the neighbours of the affected region. On the other hand, if the result of the determination S814 is negative then the indexing continues (S816) based on the existing indexing framework without reassignment of the peripheral portions of the relevant region.

Fig.8C relates to an example method that detects and responds to a situation in which a significant number or proportion of the updated data records being mapped into a particular indexing region are grouped within a sub-space of that region, which may be an indication that the region in question actually corresponds to more than one entity and the grouped records may represent one of the plural entities A simplified example of such a situation is illustrated in Fig.12I, considering the group of dots labelled Sub in region $H_3$. Once again, what constitutes a "significant" number or proportion tends to depend on the use case and various techniques (including power analysis, sample size estimation, advanced techniques for confidence interval estimation, and so on) may be used to detect the situation where a particular detected number/proportion of updated data records grouped within a particular volume of a particular indexing region is "significant" in a given context/use case.

The reference B* in Fig.8C indicates that the example method of Fig.8C may follow on from step S105 in Fig.1 (in place of the example method illustrated in Fig.2). The first processes S821 and S822 of the Fig.8C method - to obtain update data and transform updated data records into the reduced-dimension space - may be implemented in the same way as the first processes S801 and S802 in the Fig.8A and Fig.8B methods.

According to the example method of Fig.8C, statistics are gathered, for each of the indexing regions, regarding the distribution of transformed data within this region (S823). Once again, the relevant statistics may be maintained by accumulating data continuously for each region, or by other methods (e.g. evaluating the statistics in respect of updated data records in a given sliding or tumbling window defined in terms of time or in terms of a number of data records).

According to the example method of Fig.8C, the distribution of transformed updated data records within each region is examined to detect the case where a specified quantity of transformed data records are grouped in a sub-cluster (e.g. fall within a volume of a particular size); this may detect the case where the clustering of records within a sub-space of the region is significant (S824). If the result of the S824 determination is positive then, according to the example method of Fig.8C, the relevant sub-space is, from now on, associated with a new entity different from the entity represented by the remainder of the region. On the other hand, if the result of the determination S824 is negative then the indexing continues (S826) based on the existing indexing framework.

Entity-resolution methods according to the present disclosure may incorporate the example methods of Figs.2, 7, 8A, 8B and 8C individually, all together or in different sub-combinations. It will be noted that the example methods of Figs.7, 8A, 8B and 8C may all be implemented in a system that counts the numbers of transformed updated data records located at specified locations in the reduced-dimension (indexing) space.

In an entity-resolution method that incorporates the example methods of Fig.8A and Fig.8B the situation may arise that at a given time the statistics for a given region satisfy a condition that would trigger a virtual merge with an adjacent region at the same time as satisfying a condition that would lead to reassignment of portions of the given region to its neighbours. Rules may be specified to govern what happen in such a situation. For example, a rule may specify that, in such a situation, the virtual merge will be performed in preference to reassignment of region portions.

Fig.9 illustrates a non-transitory machine-readable storage medium 10 according to an example of the present disclosure. The example non-transitory machine-readable storage medium stores machine-readable instructions 20 that may be executed by a processor 50. The arrow Q in Fig.9 represents supply (e.g. in a read operation) of the machine-readable instructions to the processor 50 for execution. The machine-readable instructions 20 may be such as to cause the processor to implement any one (or more) of the example methods of the present disclosure.

The non-transitory machine-readable storage medium 10 of Fig.9 may represent any memory accessible to processor 50 that can be used to store and retrieve data. The non-transitory machine-readable storage medium 10 may comprise random access memory (RAM), read-only memory (ROM), electrically-erasable programmable read-only memory (EEPROM), cache memory, floppy disks, hard disks, optical disks, tapes, solid state drives, flash drives, portable compact disks, and/or other storage media for storing computer-executable instructions and/or data. In some examples, the processor 50 may access non-transitory machine-readable storage medium 10 locally or remotely via a network.

In some examples, the non-transitory machine-readable storage medium 10 may be any electronic, magnetic, optical, or other physical storage device that contains or stores executable instructions. In some implementations, the non-transitory machine-readable storage medium 10 may be a non-transitory storage medium, where the term "non-transitory" does not encompass transitory propagating signals. The non-transitory machine-readable storage medium 10 may be implemented in a single device or distributed across devices. Likewise, processor 50 may represent any number of processors capable of executing instructions stored by the machine-readable storage medium. The processor 50 may be integrated in a single device or distributed across devices. Further, the machine-readable storage medium 10 may be fully or partially integrated in the same device as the processor 50, or it may be separate but accessible to that device and the processor 50.

In one example, the machine-readable storage medium 10 may comprise instructions that may be part of an installation package that when installed can be executed by processor 50 to implement the functionality described herein. For example, the machine-readable storage medium may be a portable medium such as a floppy disk, CD, DVD, or flash drive or a memory maintained by a server from which the installation package can be downloaded and installed. In another example, the program instructions may be part of an application or applications already installed. Here, the machine-readable storage medium may include a hard disk, optical disk, tapes, solid state drives, RAM, ROM, EEPROM, or the like.

The processor 50 may be at least one central processing unit (CPU), microprocessor, and/or other hardware device suitable for retrieval and execution of instructions stored in the machine-readable storage medium. The processor 50 may fetch, decode, and execute program instructions. As an alternative or in addition to retrieving and executing instructions, the processor 50 may include at least one electronic circuit comprising a number of electronic components for performing the functionality of at least one of instructions.

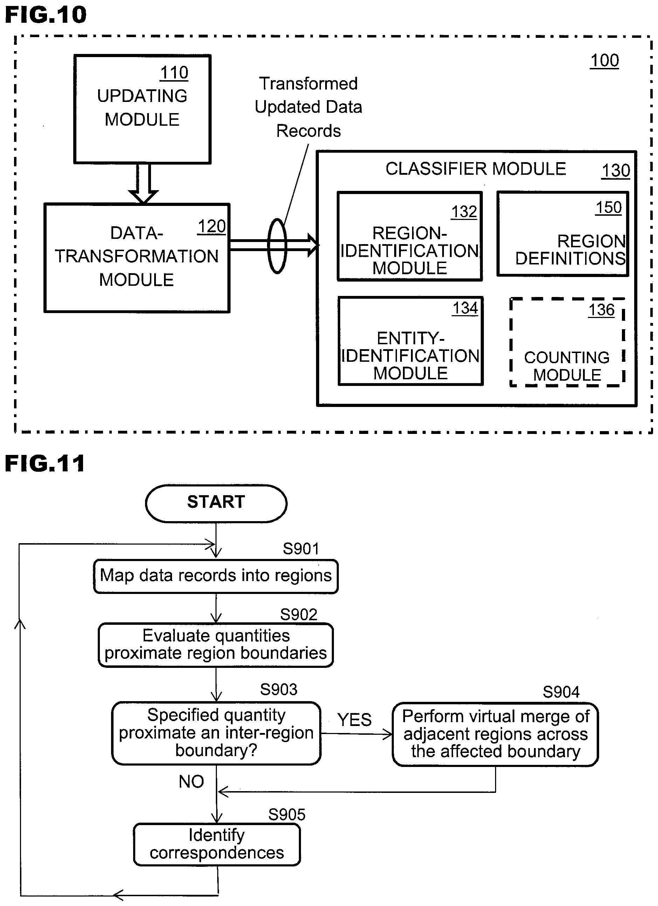

Fig.10 illustrates an automated entity-resolution system 100 that may implement example entity-resolution methods according to the present disclosure. The automated entity-resolution system 100 includes a classifier module 130 to identify the correspondence between different entities and data records of a data set. The classifier module 130 stores definitions 150 of the respective indexing regions. These definitions may be in the form of explicit data that is stored or may be provided in another manner. For example, the region definitions 150 may be inherent in a set of classifiers which implement a classification of points within the indexing space into said regions.

The automated entity-resolution system 100 further includes an updating module 110, and a data-transformation module 120. The updating module 110 supplies update data to add, modify or delete data records of the data-set. The data-transformation module transforms updated data records into the indexing space by application of the selected dimensionality-reduction method to plural attributes of the updated data records.

The updating module 110 may take different forms depending on the application. For example, in a case where entity-resolution is being performed on a streaming data set such as a Twitter feed®, the updating module 110 may be a buffering module that controls the inputting of tweets from a data source (not shown) to the automated entity-resolution system 100. As another example, in a case where entity-resolution is being performed on the fly during inputting of data to a database, the updating module 110 may comprise a user interface being operated to interact with the database (not shown).

In the automated entity-resolution system 100 according to the example of Fig.10 the classifier module 130 comprises a region-identification module 132 for determining the respective locations of the transformed updated data records in the multidimensional indexing space, and for determining which of the indexing regions contain(s) the respective locations. The classifier module 130 also comprises an entity identification module 134 for determining the correspondence between entities and updated data records based on: the region which contains the location of the transformed updated data record, and on an assignment of entities to the regions in the multidimensional space.

The classifier module 130 may further comprise a counting module 136 for evaluating the numbers of transformed updated data records that are located proximate the center and proximate the boundaries of the different indexing regions, and the entity identification module 134 may be responsive to the evaluation made by the counting module 136 to change the assignment of entities to said regions in the multidimensional space responsive to certain specified criteria being satisfied (e.g. as in the methods of Figs.8A and 8B). Furthermore, the region identification module 132 may be responsive to the evaluation made by the counting module 136 to recalculate the indexing framework including the region definitions 150 (e.g. as in the example method of Fig.7 described above).

The counting module 136 may be arranged to exclude old data records from its evaluations, e.g. taking into account only the transformed updated data records in a sliding or tumbling window (defined in terms of time or a number of data records).

In some implementations, the classifier module 130 of the automated entity-resolution system 100 may itself generate the indexing framework (e.g. determine the dimensionality-reduction method, identify the indexing regions) for example according to the method of Fig.1. In other implementations the classifier module 130 may be provided with the region definitions by an external unit (not shown) and may invoke the external unit to recalculate the indexing framework when required.

According to various implementations, the automated entity-resolution system 100 of Fig.10 and the various components described herein may be implemented in hardware and/or a combination of hardware and programming that configures hardware. The term "module", as used herein, refers to a combination of hardware and programming that performs a designated function. The hardware of each module, for example, may include one or both of a processor (e.g., processor 50) and a machine-readable storage medium (e.g. medium 10), while the programming is instructions or code stored on the machine-readable storage medium and executable by the processor to perform the designated function. Furthermore, in FIG. 10 and other Figures described herein, different numbers of components, modules, or units than depicted may be used.

Fig.11 is a flow diagram which illustrates another example automated entity-resolution method according to the present disclosure. In the example method of Fig.11, update data of a dynamically-updating data set is obtained and the update data defines at least one change that may be: addition of a new data record to the data-set, modification of a data record in the data-set, or deletion of a data record from the data-set. Updated data records of the data-set (i.e. the data records affected by the update) are mapped into different regions of a multi-dimensional space based on the attributes of the updated data records (S901). Correspondences are identified between updated data records and entities based on the regions in the multidimensional space that contain the mapped updated data records. For each of the regions, an evaluation is made of the number of updated data records mapped into said region but proximate a boundary with an adjacent region (S902). Responsive to the evaluation indicating that a specified quantity of mapped data records are proximate the boundary between a pair of adjacent regions, a virtual merge (S904) is performed of the relevant pair of regions so that updated data records mapped into either of the pair of regions is classified as corresponding to the same entity but the boundaries of the adjacent regions are unchanged and separate statistics are still maintained on the quantity of updated data records mapped into each of the adjacent regions. On the other hand, in cases in which the evaluation does not indicate that a specified quantity of mapped data records are proximate the boundary between a pair of adjacent regions, the method continues to identify correspondences between updated data records and entities (S905).

An example in which an entity-resolution method according to the present disclosure is applied to an example use case will now be described. This example application relates to de-duplication performed in the "service user" (e.g. patient) database of the UK's National Health Service (NHS), and assurance of data integrity.

Various reasons may make it difficult to match up data records in the NHS patient database with specific patients, and this can increase the risk of duplicate entries being created and the risk of data being entered in respect of the wrong patient. For example, in patient names the relationship between the position of a word within the name and the status of that word as a forename or surname can be different, bearing in mind that different cultures have different practices in this regard.

NHS staff members who enter information in the patient database have instructions that are intended to prevent duplicate data records from being created in the database. For example, in a case where a staff member intends to register a "new" patient in the database the staff member is expected to check a patient index of the database to see whether or not a data record already exists for someone having the name as the "new" patient, or a similar name, or a name in which the same forename and surname have been reversed. Nevertheless it happens that duplicate data records become created, and the database also contains duplicates as a result of the acquisition of legacy data. (The expression "duplicate" is used here to denote data records which, whether or not they are identical, relate to the same patient. The "duplicate" records may exist because of spelling mistakes in the contents of one or some fields of the data records and these mistakes may render the "duplicate" data records non-identical.)

In order to seek and eradicate duplicate data records in the patient database the NHS needs to stop its systems (e.g. go offline) for a few hours during several nights. Typically, duplicate data records are identified by generating reports of data records which have the same data in certain selected fields (e.g. NHS number, date of birth, last name, first name, address, etc.). Eradication of duplicates can take different forms, e.g. deletion of one data record, inactivation of a data record, merging of data records, and so on. This process for finding and eradicating duplicate data records is slow and labour intensive.

Example entity-resolution methods according to the present disclosure may be applied to enable de-duplication in an NHS patient database and one example implementation will now be described.

In the example implementation a classifier that makes use of an indexing space of reduced dimensions may be set up according to the process of Fig.1, using a group of data records from the patient database. Measures that may enhance data integrity and may prevent data-record duplication, based on use of the classifier and its indexing space, may be implemented in an ongoing fashion as NHS staff members use the patient database (see below).

In this example implementation, during classifier set-up according to the method of Fig.1, the clusters of data records may be determined using weighted-string similarity. For example, for each data record in the group a weighted string may be formed using the values of N attributes. Then the weighted strings may be clustered (in a space having a number N of dimensions that corresponds to the number of attributes represented in the weighted strings). The clustering may be performed for example by calculation of Jaccard index values (also known as Jaccard similarity coefficients), for instance based on the attributes "name", "address", "age", "NHS patient number" and "doctor", i.e. N=5. In this application each cluster is expected to correspond to a different patient.

In this example implementation, it may be decided, for example by explicit choice made by the system designer, or automatically (e.g. via a machine learning process), that principal components analysis (PCA) is to be used as the dimensionality-reduction technique. Application of PCA may determine that the attributes "name", "address" and "age" are the most relevant attributes for defining a reduced-dimension space in which the different clusters are well-separated from one another, in which case the reduced-dimension space has a number of dimensions D that equals 3.

In this example implementation, when an NHS staff member inputs data in a patient database system equipped to implement the entity-resolution method according to the present example, the input data may be treated as an updated data record. The system may transform the input patient data (i.e. the updated data record) into the reduced-dimension space (taking into account only the patient's name, address and age) and compare the location of the transformed data with the cluster centroid positions in the reduced-space.

If the location of the transformed data for the updated data record is close to a cluster centroid in the reduced-dimension space then it is likely that the NHS staff member is inputting data relating to an existing patient already registered in the patient database. Thus measures may be taken to prevent the NHS staff member from creating a duplicate data record for this patient (e.g. an appropriate informational message may be displayed on a display screen to the NHS staff member, a processor running database manager software may prevent creation of a new data record automatically, etc.). In a similar way, measures may be taken to ensure that the data that the NHS staff member is inputting becomes recorded in the database in respect of the correct patient.

If the location of the transformed updated data record in the reduced-dimension space is not close to the centroid of a cluster then this may indicate an undesired lack of certainty regarding the identity of the patient. (In view of the fact that there should be a relationship between specific patients and their data records, in the present example implementation it may be undesirable for transformed data to lie near the boundary between two regions). In such a case a notification may be given to the NHS staff member to prompt the staff member to obtain extra information to resolve the uncertainty regarding patient identity. For example, a message could be displayed to the staff member, on a display screen, saying "Did you mean X, Y or Z patients instead?". The prompt may lead the staff member to realise that a typographical error has been made or that another factor causing ambiguity may be present (e.g. use of a foreign naming schema) and may input corrected data on the spot. However, it is also possible that the relevant updated data record relates to a new patient having, for example, the same name as an existing patient. In such a case the staff member may be offered the possibility of defining within an existing indexing region, a new sub-cluster corresponding to the new patient, The "adaptivity" of the system makes it possible to create a subcluster within the relevant hyperspace "cell" and remember that part of the hypergrid is subdivided. In this case merge and reassignment may also be manually supervised by the staff member, with the system only suggesting possible options.

The above-described streaming approach may eliminate NHS downtime and may make staff members creating a new record aware that they are actually making a mistake or that the patient could already be in the system. Detection of likely error at the time of data input, when the patient may still be present, may be beneficial because the patient can be asked for information to solve ambiguity or avoid mistakes.

Although the present document describes various implementations of example methods, systems and computer-readable media for performing automated entity-resolution, it will be understood that the present disclosure is not limited by reference to the details of the specific implementations and variations and adaptations may be made within the scope of the appended claims.

For example, features of the various example methods may be combined with one another in substantially any combinations and sub-combinations.

The above description refers to examples in which the input data records are analysed to determine whether or not they relate to a common subject. There may be contexts in which data records have more than one type of subject. For example: on Twitter®, tweets might mention a person and an event and in a given application it might be desired to be able to identify tweets that relate to the same person as well as being able to identify tweets that relate to the same event. Example methods, non-transitory computer-readable media and systems according to the present disclosure may be applied in contexts where the data records being processed have more than one type of subject. In such contexts, one approach may involve applying a separate entity resolution process to analyse data records with reference to each type of subject. For instance, a first entity-resolution process may analyse tweets by reference to the people they mention and a second entity resolution process may analyse tweets by reference to places they mention, and so on.

An example application of entity resolution methods according to examples of the present disclosure has been described above in the context of performing "deduplication" in a database. However, the methods may be sued in numerous other applications.

Entity resolution methods according to examples of the present disclosure may be applied, for example, to facilitate merging of two, three or more than three data sets from different sources that may be organized according to different schema (e.g. to facilitate merging of databases that may not have all the same fields). In response to an example entity-resolution method assigning data records from different data sources to a portion of the reduced-dimension space that is associated with a single entity, the data of the two different data records may be merged based on an assumption that all the information in the two data records relates to the same entity.

Entity resolution methods according to examples of the present disclosure may be applied, for example, to enable identification of subjects that are trending in messages, tweets, online discussions and so on. In response to an example entity-resolution method assigning different data records to various regions defined in the reduced-dimension space, the relative numbers of data records assigned to particular regions (and, hence, relating to different particular subjects), may be evaluated.

## Claims

1. A non-transitory computer-readable medium with machine-readable instructions stored thereon that, when executed by a processor:

   obtain (S101) a group of data records ($n^0$ to $n^9$) of a data-set;
   assess similarity (S102) between data records ($n^0$ to $n^9$) in the group, based on a number N of plural attributes of said data records;
   identify clusters ($Cl_1$, $Cl_2$) of similar data records (S103) in the group based on the assessed similarity;
   determine, in a multidimensional space having a number D of dimensions less than the number N, respective regions (S104) ($H_1$ to $H_4$) corresponding to different clusters ($Cl_1$, $Cl_2$) determined by the identifying, wherein a selected dimensionality-reduction method transforms data records ($n^0$ to $n^9$) into said multidimensional space; and
   set up a classifier (S105) to identify correspondences between entities and updated data records compared to said group, based on the regions ($H_1$ to $H_4$) in said multidimensional space that contain said updated data records after transformation thereof according to the selected dimensionality-reduction method; wherein, in the similarity evaluation, similar data records ($n^0$ to $n^9$) are identified using an unsupervised similarity-evaluation method and, in the cluster identification, clusters are identified using an unsupervised cluster-identification method; further comprising instructions to:

> evaluate, for each region ($H_1$ to $H_4$), the quantity of data records ($n^0$ to $n^9$) whose transformed data is determined to be within the region but proximate a boundary with an adjacent region (S803); and
> in a case in which the evaluation indicates that the transformed data of a specified quantity of data records ($n^0$ to $n^9$) are proximate the boundary between a pair of adjacent regions ($H_1$ to $H_4$), perform a virtual merge (S805) of said pair of regions so that data records whose transformed data is determined to be in either of the pair of regions is classified as corresponding to the same entity but the boundaries of the adjacent regions are unchanged and separate counts are maintained of the numbers of data records whose transformed data is in each of the adjacent regions.

2. The non-transitory computer-readable medium according to claim 1, wherein the data-set ($n^0$ to $n^9$) comprises streaming data and said evaluation takes into account a windowed set of the most recent data records of the stream.

3. An automated entity resolution system comprising a processor configured, in response to machine-readable instructions, to:

> obtain (S101) a group of data records ($n^0$ to $n^9$) of a data-set;
> assess similarity (S102) between data records ($n^0$ to $n^9$) in the group, based on a number N of plural attributes of said data records;
> identify clusters ($Cl_1$, $Cl_2$) of similar data records (S103) in the group based on the assessed similarity;
> determine, in a multidimensional space having a number D of dimensions less than the number N, respective regions (S104) ($H_1$ to $H_4$) corresponding to different clusters ($Cl_1$, $Cl_2$) determined by the identifying, wherein a selected dimensionality-reduction method transforms data records ($n^0$ to $n^9$) into said multidimensional

space; and
set up a classifier (S105) to identify correspondences between entities and updated data records compared to said group, based on the regions ($H_1$ to $H_4$) in said multidimensional space that contain said updated data records after transformation thereof according to the selected dimensionality-reduction method; wherein, in the similarity evaluation, similar data records ($n^0$ to $n^9$) are identified using an unsupervised similarity-evaluation method and, in the cluster identification, clusters are identified using an unsupervised cluster-identification method; the processor being further configured to:

> evaluate, for each region ($H_1$ to $H_4$), the quantity of data records ($n^0$ to $n^9$) whose transformed data is determined to be within the region but proximate a boundary with an adjacent region (S803); and
> in a case in which the evaluation indicates that the transformed data of a specified quantity of data records ($n^0$ to $n^9$) are proximate the boundary between a pair of adjacent regions ($H_1$ to $H_4$), perform a virtual merge (S805) of said pair of regions so that data records whose transformed data is determined to be in either of the pair of regions is classified as corresponding to the same entity but the boundaries of the adjacent regions are unchanged and separate counts are maintained of the numbers of data records whose transformed data is in each of the adjacent regions.

4. The system of claim 3, wherein the data-set ($n^0$ to $n^9$) comprises streaming data and said evaluation takes into account a windowed set of the most recent data records of the stream.

5. An automated entity resolution method, comprising:

> obtaining (S101) a group of data records ($n^0$ to $n^9$) of a data-set;
> assessing similarity (S102) between data records ($n^0$ to $n^9$) in the group, based on a number N of plural attributes of said data records;
> identify clusters ($Cl_1$, $Cl_2$) of similar data records (S103) in the group based on the assessed similarity;
> determining, in a multidimensional space having a number D of dimensions less than the number N, respective regions (S104) ($H_1$ to $H_4$) corresponding to different clusters ($Cl_1$, $Cl_2$) determined by the identifying, wherein a selected dimensionality-reduction method transforms data

records ($n^0$ to $n^9$) into said multidimensional space; and

setting up a classifier (S105) to identify correspondences between entities and updated data records compared to said group, based on the regions ($H_1$ to $H_4$) in said multidimensional space that contain said updated data records after transformation thereof according to the selected dimensionality-reduction method;

wherein, in the similarity evaluation, similar data records ($n^0$ to $n^9$) are identified using an unsupervised similarity-evaluation method and, in the cluster identification, clusters are identified using an unsupervised cluster-identification method;

evaluating, for each region ($H_1$ to $H_4$), the quantity of data records ($n^0$ to $n^9$) whose transformed data is determined to be within the region but proximate a boundary with an adjacent region (S803); and

in a case in which the evaluation indicates that the transformed data of a specified quantity of data records ($n^0$ to $n^9$) are proximate the boundary between a pair of adjacent regions ($H_1$ to $H_4$), performing a virtual merge of said pair of regions so that data records whose transformed data is determined to be in either of the pair of regions is classified as corresponding to the same entity but the boundaries of the adjacent regions are unchanged and separate counts are maintained of the numbers of data records whose transformed data is in each of the adjacent regions (S805).

6. The method of claim 5, wherein the data-set ($n^0$ to $n^9$) comprises streaming data and said evaluation takes into account a windowed set of the most recent data records of the stream.

**Patentansprüche**

1. Nichtflüchtiges computerlesbares Medium mit darauf gespeicherten maschinenlesbaren Anweisungen, die, wenn sie durch einen Prozessor ausgeführt werden, Folgendes veranlassen:

Erhalten (S101) einer Gruppe von Datensätzen ($n^0$ bis $n^9$) einer Datengruppe;
Beurteilen der Ähnlichkeit (S102) zwischen Datensätzen ($n^0$ bis $n^9$) in der Gruppe basierend auf einer Anzahl N von mehreren Attributen der Datensätze;
Identifizieren von Clustern ($Cl_1$, $Cl_2$) ähnlicher Datensätze (S103) in der Gruppe basierend auf der beurteilten Ähnlichkeit;
Bestimmen, in einem mehrdimensionalen Raum mit einer Anzahl D von Dimensionen kleiner als die Anzahl N, jeweiliger Bereiche (S104) ($H_1$ bis $H_4$), die verschiedenen Clustern ($Cl_1$, $Cl_2$) entsprechen, die durch das Identifizieren bestimmt werden, wobei ein ausgewähltes Dimensionsreduzierungsverfahren Datensätze ($n^0$ bis $n^9$) in den mehrdimensionalen Raum umwandelt; und

Einrichten eines Klassifizierers (S105), um Entsprechungen zwischen Entitäten und aktualisierten Datensätzen im Vergleich zu der Gruppe basierend auf den Regionen ($H_1$ bis $H_4$) in dem mehrdimensionalen Raum zu identifizieren, die die aktualisierten Datensätze nach einer Umwandlung davon gemäß dem ausgewählten Dimensionsreduzierungsverfahrens enthalten;

wobei in der Ähnlichkeitsauswertung ähnliche Datensätze ($n^0$ bis $n^9$) unter Verwendung eines nichtüberwachten Ähnlichkeitsauswertungsverfahrens identifiziert werden und in der Clusteridentifizierung Cluster unter Verwendung eines nichtüberwachten Clusteridentifizierungsverfahrens identifiziert werden;

ferner umfassend Anweisungen für Folgendes:

Auswerten, für jeden Bereich ($H_1$ bis $H_4$), der Quantität der Datensätze ($n^0$ bis $n^9$), deren umgewandelten Daten als innerhalb des Bereichs aber nahe einer Abgrenzung zu einem benachbarten Bereich (S803) befindlich bestimmt werden; und

in einem Fall, in dem die Auswertung anzeigt, dass die umgewandelten Daten einer bestimmten Quantität von Datensätzen ($n^0$ bis $n^9$) nahe der Abgrenzung zwischen einem Paar benachbarter Bereiche ($H_1$ bis $H_4$) sind, Durchführen eines virtuellen Zusammenführens (S805) des Paares von Bereichen, sodass Datensätze, deren umgewandelte Daten als in einem des Paars von Bereichen befindlich bestimmt werden, als der gleichen Entität entsprechend klassifiziert werden, die Grenzen der benachbarten Bereiche jedoch unverändert sind und getrennte Zählwerte der Anzahlen von Datensätzen, deren umgewandelte Daten sich in jedem der benachbarten Bereiche befinden, beibehalten werden.

2. Nichtflüchtiges computerlesbares Medium nach Anspruch 1, wobei die Datengruppe ($n^0$ bis $n^9$) Streaming-Daten umfasst und die Auswertung einen Fenstermodussatz der neuesten Datensätze des Stroms berücksichtigt.

3. Automatisiertes Entitätenauflösungssystem, das einen Prozessor umfasst, der als Reaktion auf maschinenlesbare Anweisungen für Folgendes konfiguriert ist:

Erhalten (S101) einer Gruppe von Datensätzen ($n^0$ bis $n^9$) einer Datengruppe;

Beurteilen der Ähnlichkeit (S102) zwischen Datensätzen ($n^0$ bis $n^9$) in der Gruppe basierend auf einer Anzahl N von mehreren Attributen der Datensätze;

Identifizieren von Clustern ($Cl_1$, $Cl_2$) ähnlicher Datensätze (S103) in der Gruppe basierend auf der beurteilten Ähnlichkeit;

Bestimmen, in einem mehrdimensionalen Raum mit einer Anzahl D von Dimensionen kleiner als die Anzahl N, jeweiliger Bereiche (S104) ($H_1$ bis $H_4$), die verschiedenen Clustern ($Cl_1$, $Cl_2$) entsprechen, die durch das Identifizieren bestimmt werden, wobei ein ausgewähltes Dimensionsreduzierungsverfahren Datensätze ($n^0$ bis $n^9$) in den mehrdimensionalen Raum umwandelt; und

Einrichten eines Klassifizierers (S105), um Entsprechungen zwischen Entitäten und aktualisierten Datensätzen im Vergleich zu der Gruppe basierend auf den Regionen ($H_1$ bis $H_4$) in dem mehrdimensionalen Raum zu identifizieren, die die aktualisierten Datensätze nach einer Umwandlung davon gemäß dem ausgewählten Dimensionsreduzierungsverfahrens enthalten;

wobei in der Ähnlichkeitsauswertung ähnliche Datensätze ($n^0$ bis $n^9$) unter Verwendung eines nichtüberwachten Ähnlichkeitsauswertungsverfahrens identifiziert werden und in der Clusteridentifizierung Cluster unter Verwendung eines nichtüberwachten Clusteridentifizierungsverfahrens identifiziert werden;

wobei der Prozessor ferner für Folgendes konfiguriert ist:

Auswerten, für jeden Bereich ($H_1$ bis $H_4$), der Quantität der Datensätze ($n^0$ bis $n^9$), deren umgewandelte Daten als innerhalb des Bereichs aber nahe einer Abgrenzung zu einem benachbarten Bereich (S803) befindlich bestimmt werden; und

in einem Fall, in dem die Auswertung anzeigt, dass die umgewandelten Daten einer bestimmten Quantität von Datensätzen ($n^0$ bis $n^9$) nahe der Abgrenzung zwischen einem Paar benachbarter Bereiche ($H_1$ bis $H_4$) sind, Durchführen eines virtuellen Zusammenführens (S805) des Paares von Bereichen, sodass Datensätze, deren umgewandelte Daten als in einem des Paars von Bereichen befindlich bestimmt werden, als der gleichen Entität entsprechend klassifiziert werden, die Grenzen der benachbarten Bereiche jedoch unverändert sind und getrennte Zählwerte der Anzahlen von Datensätzen, deren umgewandelte Daten sich in jedem der benachbarten Bereiche befinden, beibehalten werden.

4. System nach Anspruch 3, wobei die Datengruppe ($n^0$ bis $n^9$) Streaming-Daten umfasst und die Auswertung einen Fenstermodussatz der neuesten Datensätze des Stroms berücksichtigt.

5. Automatisiertes Entitätsauflösungsverfahren, Folgendes umfassend:

Erhalten (S101) einer Gruppe von Datensätzen ($n^0$ bis $n^9$) einer Datengruppe;

Beurteilen der Ähnlichkeit (S102) zwischen Datensätzen ($n^0$ bis $n^9$) in der Gruppe basierend auf einer Anzahl N von mehreren Attributen der Datensätze;

Identifizieren von Clustern ($Cl_1$, $Cl_2$) ähnlicher Datensätze (S103) in der Gruppe basierend auf der beurteilten Ähnlichkeit;

Bestimmen, in einem mehrdimensionalen Raum mit einer Anzahl D von Dimensionen kleiner als die Anzahl N, jeweiliger Bereiche (S104) ($H_1$ bis $H_4$), die verschiedenen Clustern ($Cl_1$, $Cl_2$) entsprechen, die durch das Identifizieren bestimmt werden, wobei ein ausgewähltes Dimensionsreduzierungsverfahren Datensätze ($n^0$ bis $n^9$) in den mehrdimensionalen Raum umwandelt; und

Einrichten eines Klassifizierers (S105), um Entsprechungen zwischen Entitäten und aktualisierten Datensätzen im Vergleich zu der Gruppe basierend auf den Regionen ($H_1$ bis $H_4$) in dem mehrdimensionalen Raum zu identifizieren, die die aktualisierten Datensätze nach einer Umwandlung davon gemäß dem ausgewählten Dimensionsreduzierungsverfahrens enthalten;

wobei in der Ähnlichkeitsauswertung ähnliche Datensätze ($n^0$ bis $n^9$) unter Verwendung eines nichtüberwachten Ähnlichkeitsauswertungsverfahrens identifiziert werden und in der Clusteridentifizierung Cluster unter Verwendung eines nichtüberwachten Clusteridentifizierungsverfahrens identifiziert werden;

Auswerten, für jeden Bereich ($H_1$ bis $H_4$), der Quantität der Datensätze ($n^0$ bis $n^9$), deren umgewandelte Daten als innerhalb des Bereichs aber nahe einer Abgrenzung zu einem benachbarten Bereich (S803) befindlich bestimmt werden; und

in einem Fall, in dem die Auswertung anzeigt, dass die umgewandelten Daten einer bestimmten Quantität von Datensätzen ($n^0$ bis $n^9$) nahe der Abgrenzung zwischen einem Paar benachbarter Bereiche ($H_1$ bis $H_4$) sind, Durchführen eines virtuellen Zusammenführens des Paares von Bereichen, sodass Datensätze, deren umgewandelte Daten als in einem des Paars von Bereichen befindlich bestimmt werden, als der gleichen Entität entsprechend klassifiziert werden, die Grenzen der benachbarten Bereiche jedoch unverändert sind und getrennte Zählwerte der Anzahlen von Datensätzen, deren umgewandelte Daten sich in jedem der benachbarten Bereiche (S805) befinden, beibehalten werden.

**6.** Verfahren nach Anspruch 5, wobei die Datengruppe ($n^0$ bis $n^9$) Streaming-Daten umfasst und die Auswertung einen Fenstermodussatz der neuesten Datensätze des Stroms berücksichtigt.

**Revendications**

**1.** Support non transitoire lisible par ordinateur sur lequel sont stockées des instructions lisibles par machine qui, lorsqu'elles sont exécutées par un processeur :

obtiennent (S101) un groupe d'enregistrements de données ($n^0$ à $n^9$) d'un ensemble de données ;
évaluent la similarité (S102) entre des enregistrements de données ($n^0$ à $n^9$) dans le groupe, sur la base d'un nombre N de plusieurs attributs desdits enregistrements de données ;
identifient des groupements ($Cl_1$, $Cl_2$) d'enregistrements de données similaires (S103) dans le groupe sur la base de la similarité évaluée ;
déterminent, dans un espace multidimensionnel ayant un nombre D de dimensions inférieur au nombre N, des régions respectives (S104) ($H_1$ à $H_4$) correspondant à différents groupements ($Cl_1$, $Cl_2$) déterminés par l'identification, un procédé de réduction de dimensionnalité sélectionné transformant les enregistrements de données ($n^0$ à $n^9$) dans ledit espace multidimensionnel ; et
établissent un classificateur (S105) pour identifier des correspondances entre des entités et des enregistrements de données mis à jour comparés audit groupe, sur la base des régions ($H_1$ à $H_4$) dans ledit espace multidimensionnel qui contiennent lesdits enregistrements de données mis à jour après leur transformation conformément au procédé de réduction de dimensionnalité sélectionné ;
dans l'évaluation de similarité, des enregistrements de données similaires ($n^0$ à $n^9$) étant identifiés en utilisant un procédé d'évaluation de similarité non supervisé et, dans l'identification de groupement, des groupements étant identifiés en utilisant un procédé d'identification de groupement non supervisé ;
comprenant en outre des instructions pour :

évaluer, pour chaque région ($H_1$ à $H_4$), la

quantité d'enregistrements de données ($n^0$ à $n^9$) dont les données transformées sont déterminées comme se trouvant à l'intérieur de la région mais à proximité d'une limite avec une région adjacente (S803) ; et
dans le cas où l'évaluation indique que les données transformées d'une quantité spécifiée d'enregistrements de données ($n^0$ à $n^9$) sont proches de la limite entre une paire de régions adjacentes ($H_1$ à $H_4$), effectuer une fusion virtuelle (S805) de ladite paire de régions de sorte que les enregistrements de données dont les données transformées sont déterminées comme se trouvant dans l'une ou l'autre de la paire de régions sont classés comme correspondant à la même entité, mais que les limites des régions adjacentes restent inchangées et que des comptages séparés des nombres d'enregistrements de données dont les données transformées se trouvent dans chacune des régions adjacentes sont conservés.

**2.** Support non transitoire lisible par ordinateur selon la revendication 1, dans lequel l'ensemble de données ($n^0$ à $n^9$) comprend des données en flux continu et ladite évaluation prend en compte un ensemble à fenêtres des enregistrements de données les plus récents du flux continu.

**3.** Système de résolution d'entité automatisé comprenant un processeur configuré, en réponse à des instructions lisibles par machine, pour :

obtenir (S101) un groupe d'enregistrements de données ($n^0$ à $n^9$) d'un ensemble de données ;
évaluer la similarité (S102) entre des enregistrements de données ($n^0$ à $n^9$) dans le groupe, sur la base d'un nombre N de plusieurs attributs desdits enregistrements de données ;
identifier des groupements ($Cl_1$, $Cl_2$) d'enregistrements de données similaires (S103) dans le groupe sur la base de la similarité évaluée ;
déterminer, dans un espace multidimensionnel ayant un nombre D de dimensions inférieur au nombre N, des régions respectives (S104) ($H_1$ à $H_4$) correspondant à différents groupements ($Cl_1$, $Cl_2$) déterminés par l'identification, un procédé de réduction de dimensionnalité sélectionné transformant des enregistrements de données ($n^0$ à $n^9$) dans ledit espace multidimensionnel ; et
établir un classificateur (S105) pour identifier des correspondances entre des entités et des enregistrements de données mis à jour comparés audit groupe, sur la base des régions ($H_1$ à $H_4$) dans ledit espace multidimensionnel qui contiennent lesdits enregistrements de données mis à jour après leur transformation conformément au procédé de réduction de dimensionnalité sélectionné ;
dans l'évaluation de similarité, des enregistrements de données similaires ($n^0$ à $n^9$) étant identifiés en utilisant un procédé d'évaluation de similarité non supervisé et, dans l'identification de groupement, des groupements étant identifiés en utilisant un procédé d'identification de groupement non supervisé ;
le processeur étant en outre configuré pour :

évaluer, pour chaque région ($H_1$ à $H_4$), la quantité d'enregistrements de données ($n^0$ à $n^9$) dont les données transformées sont déterminées comme se trouvant à l'intérieur de la région mais à proximité d'une limite avec une région adjacente (S803) ; et
dans le cas où l'évaluation indique que les données transformées d'une quantité spécifiée d'enregistrements de données ($n^0$ à $n^9$) sont proches de la limite entre une paire de régions adjacentes ($H_1$ à $H_4$), effectuer une fusion virtuelle (S805) de ladite paire de régions de sorte que les enregistrements de données dont les données transformées sont déterminées comme se trouvant dans l'une ou l'autre de la paire de régions sont classés comme correspondant à la même entité, mais que les limites des régions adjacentes restent inchangées et que des comptages séparés des nombres d'enregistrements de données dont les données transformées se trouvent dans chacune des régions adjacentes sont conservés.

4. Système selon la revendication 3, dans lequel l'ensemble de données ($n^0$ à $n^9$) comprend des données en flux continu et ladite évaluation prend en compte un ensemble à fenêtres des enregistrements de données les plus récents du flux continu.

5. Procédé de résolution d'entité automatisé, comprenant :

l'obtention (S101) d'un groupe d'enregistrements de données ($n^0$ à $n^9$) d'un ensemble de données ;
l'évaluation de la similarité (S102) entre des enregistrements de données ($n^0$ à $n^9$) dans le groupe, sur la base d'un nombre N de plusieurs attributs desdits enregistrements de données ;
l'identification de groupements ($Cl_1$, $Cl_2$) d'enregistrements de données similaires (S103) dans le groupe sur la base de la similarité évaluée ;
la détermination, dans un espace multidimensionnel ayant un nombre D de dimensions inférieur au nombre N, de régions respectives (S104) ($H_1$ à $H_4$) correspondant à différents groupements ($Cl_1$, $Cl_2$) déterminés par l'identification, un procédé de réduction de dimensionnalité sélectionné transformant des enregistrements de données ($n^0$ à $n^9$) dans ledit espace multidimensionnel ; et
l'établissement d'un classificateur (S105) pour identifier des correspondances entre des entités et des enregistrements de données mis à jour comparés audit groupe, sur la base des régions ($H_1$ à $H_4$) dans ledit espace multidimensionnel qui contiennent lesdits enregistrements de données mis à jour après leur transformation conformément au procédé de réduction de dimensionnalité sélectionné ;
dans l'évaluation de similarité, des enregistrements de données similaires ($n^0$ to $n^9$) étant identifiés en utilisant un procédé d'évaluation de similarité non supervisé et, dans l'identification de groupement, des groupements étant identifiés en utilisant un procédé d'identification de groupement non supervisé ;
l'évaluation, pour chaque région ($H_1$ à $H_4$), de la quantité d'enregistrements de données ($n^0$ to $n^9$) dont les données transformées sont déterminées comme se trouvant à l'intérieur de la région mais à proximité d'une limite avec une région adjacente (S803) ; et
dans le cas où l'évaluation indique que les données transformées d'une quantité spécifiée d'enregistrements de données ($n^0$ à $n^9$) sont proches de la limite entre une paire de régions adjacentes ($H_1$ à $H_4$), la réalisation d'une fusion virtuelle de ladite paire de régions de sorte que les enregistrements de données dont les données transformées sont déterminées comme se trouvant dans l'une ou l'autre de la paire de régions sont classés comme correspondant à la même entité, mais que les limites des régions adjacentes restent inchangées et que des comptages séparés des nombres d'enregistrements de données dont les données transformées se trouvent dans chacune des régions adjacentes (S805) sont conservés.

6. Procédé selon la revendication 5, dans lequel l'ensemble de données ($n^0$ à $n^9$) comprend des données en flux continu et ladite évaluation prend en compte un ensemble à fenêtres des enregistrements de données les plus récents du flux continu.

**FIG.1**

A

S101
Obtain group of data records

S102
Evaluate similarity of data records in group

S103
Identify clusters

S104
Determine regions in reduced-dimension space

S105
Set-up classifier for updated data records

B

**FIG.2**

B

S201
Receive update data

S202
Transform updated data records

S203
Identify correspondences

**FIG.3A**

```
        ┌─────────────┐
        │    START    │
        └──────┬──────┘
               │  S301
        ┌──────▼──────┐
        │  Transform  │
        │   cluster   │
        │  centroids  │
        └──────┬──────┘
               │  S302
   ┌───────────▼───────────┐
   │    Compute region     │
   │ boundaries (to separate│
   │ transformed centroids) │
   └───────────┬───────────┘
               │
        ┌──────▼──────┐
        │     END     │
        └─────────────┘
```

**FIG.3B**

```
        ┌─────────────┐
        │    START    │
        └──────┬──────┘
               │  S310
        ┌──────▼──────┐
        │  Transform  │
        │   cluster   │
        │  centroids  │
        └──────┬──────┘
               │  S311
   ┌───────────▼───────────┐
   │   Determine ranges    │
   │   associated to the   │
   │      transformed      │
   │   cluster centroids   │
   └───────────┬───────────┘
               │  S312
   ┌───────────▼───────────┐
   │    Compute region     │
   │ boundaries (to separate│
   │ transformed centroids) │
   │    – under constraint  │
   └───────────┬───────────┘
               │
        ┌──────▼──────┐
        │     END     │
        └─────────────┘
```

**FIG.4**

**FIG.5**

**FIG.6**

START (j=1)

S601
Transform centroid data, & set associated range, using jth combination of dimension-reduction method and range-determination method

j → j+1

S602
Compute region boundaries

S603
Evaluate region separation

S604
Last j ?

NO

YES

S605
Select combination giving best separation of regions

END

**FIG.7**

B'

S701
Obtain update data

S702
Transform updated data records

S703
Quantity (outside set of regions) > threshold?

YES

A

NO

S704
Identify correspondences

**FIG.8A**

B″

S801
Obtain update data

S802
Transform updated data records

S803
Evaluate quantities of transformed updated data records proximate inter-region boundaries

S804
Specified quantity proximate an inter-region boundary?

YES

S805
Perform virtual merge of adjacent regions across the affected boundary

NO

S806
Identify correspondences

**FIG.8B.**

B‴

S811
Obtain update data

S812
Transform updated data records

S813
Evaluate quantities proximate centers, boundaries

S814
For any region: No.(boundary) > No.(center)+k ?

YES

S815
Reassign border portions of affected region(s)

NO

S816
Identify correspondences

**FIG.8C**

B*

S821
Obtain update data

S822
Transform updated data records

S823
Assess distribution of transformed updated data records within each region

S824
Specified quantity grouped in a sub-cluster?

YES

S825
Define sub-cluster region

NO

S826
Identify correspondences

**FIG.9**

COMPUTER-READABLE MEDIUM

Q

MACHINE-READABLE INSTRUCTIONS 20

10

PROCESSOR

50

## FIG.10

**110**
UPDATING MODULE

Transformed Updated Data Records

**100**

DATA-TRANSFORMATION MODULE **120**

CLASSIFIER MODULE **130**

REGION-IDENTIFICATION MODULE **132**

REGION DEFINITIONS **150**

ENTITY-IDENTIFICATION MODULE **134**

COUNTING MODULE **136**

## FIG.11

START

↓ S901
Map data records into regions

↓ S902
Evaluate quantities proximate region boundaries

↓ S903
Specified quantity proximate an inter-region boundary? — YES → Perform virtual merge of adjacent regions across the affected boundary **S904**

NO

↓ S905
Identify correspondences

# FIG.12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6134541 A

**Non-patent literature cited in the description**

- **BHATTACHARYA.** *Collective Entity Resolution in Relational Data*